# EUROPEAN PATENT SPECIFICATION

(11) **EP 1 430 930 B1**
(45) Date of publication and mention of the grant of the patent: **23.08.2006**
(21) Application number: 03024990.8
(22) Date of filing: 30.10.2003
(51) Int. Cl.: A61N 1/37, A61N 1/368

(54) **An implantable heart stimulating device and a system including such a device**
Implantierbarer Herzschrittmacher und System mit einem solchem Gerät
Dispositif implantable de stimulation cardiaque et système comprenant ce dispositif

(30) Priority: 16.12.2002 SE 0203727
(43) Date of publication of application: 23.06.2004
(73) Proprietor: St. Jude Medical AB, 175 84 Järfälla (SE)
(72) Inventor: Järverud, Karin, 169 71 Solna (SE); Holmström, Nils, 175 57 Järfälla (SE); Björling, Anders, 157 53 Järfälla (SE); Uhrenius, Asa, 113 30 Stockholm (SE); Hedberg, Sven-Erik, 196 32 Kungsängen (SE); Budgifvars, Göran, 163 51 Spanga (SE); Strandberg, Hans, 172-65 Sundbyberg (SE)

(56) References cited:
- EP-A- 0 990 451
- US-A- 4 955 376
- US-B1- 6 456 881

## Description

### BACKGROUND OF THE INVENTION

### 1. Field of the invention

The present invention relates to an implantable heart stimulating device with which it is possible to stimulate both the ventricles of a heart, i.e. a bi-ventricular pacer.

The invention also relates to a system including such a device.

### 2. Description of the prior art

Several different implantable devices for stimulating a heart are known. The devices are normally able to sense the electrical activity of the heart. Some implantable devices are able to deliver stimulation pulses to both the left and right ventricles of the heart, and sometimes also to the left and right atria.

Devices that are able to deliver stimulation pulses to both the left and right ventricles are also called bi-ventricular pacers. Such devices can be used to treat patients who suffer from different severe cardiac problems, e.g. patients suffering from congestive heart failure (CHF). CHF is defined generally as the inability of the heart to deliver a sufficient amount of blood to the body. CHF can have different causes. It can for example be caused by a left bundle branch block (LBBB) or a right bundle branch block (RBBB). By using bi-ventricular pacing, the contraction of the ventricles can be controlled in order to improve the ability of the heart to pump blood. The stimulation pulses to the two ventricles can be delivered simultaneously but it is also known that the stimulation pulses to the two ventricles are delivered with a short time delay between them in order to optimise the pumping performance of the heart.

US-A-5 720 768 describes different possible electrode positions in order to stimulate or sense the different chambers of the heart.

US-A-6 070 100 describes that electrodes may be positioned in both the left and the right atrium as well as in the left and the right ventricles.

In connection with implantable pacers, in particular pacers which only have the possibility to stimulate the right ventricle, and sometimes also the right atrium, it is known to detect the capture of the heart, i.e. to detect whether the heart actually reacts to a delivered stimulation pulse. If the heart is not captured it is possible to arrange the pacer to deliver a back-up pulse with a higher pulse energy than the first pulse. It is also possible to increase the pulse energy in future stimulation pulses if capture is not detected. In order to save battery it is important that the stimulation pulses are not delivered with an unnecessarily high energy. By varying the energy of the stimulation pulses and by detecting the capture it is possible to find a threshold value for the stimulation pulse energy. Based on the threshold value, a suitable stimulation pulse energy can be determined.

The detection of capture involves several problems. Different signals from the heart or generated by the pacemaker may interfere with each other, which may make the detection of capture difficult. The evoked response that it is intended to detect may thus be hidden because of other electrical phenomena. It is particularly difficult to detect capture in a bi-ventricular pacer, since in such a pacer there are more delivered and detected signals which may interfere with each other.

US-A-6,148,234 describes a system for detecting capture in connection with bi-ventricular or bi-atrial pacing. The document describes the fact that if a chamber is captured, then there is a biological refractory period during which this chamber cannot be stimulated again. The system described in this document monitors these refractory periods for the different chambers, for example for the two ventricles. When capture is achieved in both ventricles, no intrinsic depolarisation signals can be sensed during the following refractory period. However, where the output level of one of the pacing pulses is insufficient to capture one ventricle, but capture is achieved in the other ventricle, a delayed depolarisation pattern can be detected in the ventricle that was not captured. This delayed depolarisation is due to an interventricular conduction from the ventricle that is captured to the ventricle that is not captured. The system according to this document thus monitors the refractory interval following each delivery of stimulating pulses to the ventricles. A loss of capture is indicated in case such a delayed depolarisation is sensed during the refractory period.

Also US 2001/0049542 A1 describes a system for detecting capture in connection with bi-ventricular or bi-atrial stimulation. The system includes a morphology detector incorporated in a micro controller to allow for the processing of the sensed intra-cardiac electrogram signals (IEGM). The morphology of the IEGM may depend on whether both the ventricles (or atria) have captured or not. By detecting the shape of the IEGM capture may thus be detected.

US 6,456,881 B1 describes a multi-chamber stimulation device and a method for distinguishing fusion from loss of capture during ventricular stimulation. A far-field signal present in the atrial channel is examined for evidence of a far-field R-wave whenever the ventricular channel detects a loss of capture. If such a far-field R-wave is present, fusion is confirmed. If such a far-field R-wave is absent, loss of capture is confirmed.

US 4,955,376 describes a pacemaker provided with an improved automatic regulation circuit which is capable of distinguishing between a fusion beat and loss of capture. If no evoked response signal is sensed after a stimulus, the pacemaker generates a high-energy back-up pulse shortly after the stimulation. Failure to capture by the back-up pulse is an indication that there was a fusion beat.

Another concept used in this technical field is the concept "fusion". A fusion may occur when an intrinsic depolarisation of the heart takes place simultaneously, or substantially simultaneously, with a stimulation pulse from the heart stimulating device.

### SUMMARY OF THE INVENTION

The present invention concerns in particular an implantable heart stimulating device including a control circuit comprising:
first pacing means adapted to be connected to a first pacing electrode suited to be positioned in or at a first ventricle of a heart and to receive signals from said first pacing means such that said first pacing means are able to pace said first ventricle;
first sensing means adapted to be connected to a first sensing electrode suited to be positioned in or at said first ventricle of the heart and to transfer signals to said first sensing means such that said first sensing means are able to sense said first ventricle;
second pacing means adapted to be connected to a second pacing electrode suited to be positioned in or at a second ventricle of the heart and to receive signals from said second pacing means such that said second pacing means are able to pace said second ventricle;
second sensing means adapted to be connected to a second sensing electrode suited to be positioned in or at said second ventricle of the heart and to transfer signals to said second sensing means such that said second sensing means are able to sense said second ventricle;
said control circuit and said first sensing means being arranged to be able to detect a signal typical of an evoked response to a pacing pulse delivered by said first pacing means, by sensing within a first time interval that follows after a pacing pulse delivered by said first pacing means;
said control circuit and said first sensing means also being arranged to be able to detect, within a first time window, a signal of the kind typical for an R-wave transferred from said second ventricle, or from some other part of the heart, to said first ventricle, wherein this first time window is not identical with said first time interval,
said control circuit being arranged to be able to operate with time cycles corresponding to normal heart cycles; said control circuit being arranged such that if within one such time cycle pacing pulses are delivered both by said first pacing means and by said second pacing means, then these pacing pulses are delivered at least substantially simultaneously.

It should be noted that by "substantially simultaneously" is here meant that the pacing pulses are exactly simultaneous or that there is only a short time between them. The pacing pulses ought to be delivered substantially simultaneously such that the pacing pulse delivered by the second pacing means does not interfere with the detection of an evoked response to a pacing pulse delivered by the first pacing means. It should also be noted that it is not necessary that pacing pulses are delivered both by the first pacing means and the second pacing means during every time cycle, since it is also possible that the delivery of a pacing pulse is inhibited.

In connection with the above described device it may be difficult to distinguish a loss of capture in a ventricle from a situation in which fusion occurs. In a fusion beat, some cardiac cells have already been activated by a spontaneous depolarisation taking place substantially simultaneously with a pacing pulse. Thereby, the evoked response signal can be weaker than if no fusion were the case. Consequently, the mentioned first sensing means may not detect any evoked response although the ventricle in question has been depolarised during the fusion beat.

It is an object of the present invention to provide an implantable heart stimulating device of the above defined kind with which it is possible to distinguish the situation where a real loss of capture occurs from the situation when a fusion is the case, and wherein the operation of the device is controlled in accordance therewith. A further object is to provide such a device which is relatively uncomplicated and which can be implemented by relatively simple means.

The above objects are achieved by an implantable heart monitoring device of the above described kind, in which the control circuit is arranged to carry out the following steps:
a) determine whether during a time cycle said signal typical of an evoked response to a pacing pulse delivered by said first pacing means is sensed within said first time interval,
b) determine whether during the same time cycle said signal of the kind typical for an R-wave transferred from said second ventricle, or from some other part of the heart, to said first ventricle is detected within said first time window,
wherein the control circuit is arranged such that the operation of the device depends on whether the conditions in a) and b) are fulfilled.

By determining the conditions a) and b) it is possible to determine whether a fusion has occurred. Since the operation of the device should preferably be different in the situation where a fusion occurs as opposed to the situation when a real loss occurs, the control circuit is arranged such that the operation of the device depends on whether the conditions in a) and b) are fulfilled. Thereby the operation of the device may be optimised to the actually occurring condition.

It should be noted that the concept "real loss" in this document means that the ventricle in question has not been depolarised, i. e. no capture is the case, and that also no fusion has occurred.

Preferably, the control circuit is arranged such that the device will operate in a manner L1 if it is the case both that the condition in a) is not fulfilled and that the condition in b) is fulfilled, and in another manner F1 if it is the case both that the condition in a) is not fulfilled and that the condition in b) is not fulfilled. Advantageously, the manner L1 means that the device functionally operates as if a real loss of capture has occurred in the first ventricle and such that the manner F1 means that the device functionally operates as if a fusion has occurred in said first ventricle. If the condition in a) is not fulfilled, this means that no evoked response has been detected by the first sensing means. This can be due to either a real loss of capture or to a fusion. If the situation in b) is fulfilled, this means that a transferred R-wave has been detected by the first sensing means. This only occurs if the first ventricle was not depolarised. Consequently, this situation indicates that a real loss of capture has occurred. Analogously, in case no such transferred R-wave is detected, this indicates that probably a fusion is the case. It should be noted that the labels L1 and F1 are used only to distinguish the manners from each other.

According to one advantageous embodiment, if a real loss of capture (i.e. L1) has been determined a predetermined number of times, then the control circuit is arranged to vary the energy of the pacing pulses delivered by said first pacing means and to detect, with said first sensing means, signals typical for evoked responses during said first time interval such that a suitable pulse energy for the pacing pulses delivered by said first pacing means is determined. If a real loss is the case, it is thus advantageous to do a threshold search in order to determine a suitable pulse energy.

According to another embodiment, at least if a fusion (i.e. F1) has been determined a predetermined number of times, then the control circuit is arranged to modify at least one time period that controls the operation of the device. This time period can be either increased or decreased. One possibility is thus to increase the time period. If the time period is increased, then a possible stimulation pulse is delivered later. Thereby, the stimulation pulse is less likely to interfere with the intrinsic depolarisation, i. e. the risk for a fusion decreases. Analogously, it is possible to decrease the time period such that a possible stimulation pulse is delivered earlier. Also in this case the risk for a fusion decreases.

The mentioned first time interval can start 0-30ms after the delivery of a pacing pulse by said first pacing means and can be between 25ms and 100ms long. The mentioned first time window can start between 0 ms and 150 ms after the delivery of said pacing pulse by said first pacing means. The first time window preferably ends at least before 400ms after the delivery of said pacing pulse by said first pacing means. The mentioned times have been found to be suitable for detecting an evoked response and a transferred R-wave, respectively. According to an advantageous embodiment, the first time window does not start before the end of the first time interval.

According to another preferred embodiment, the control circuit is also arranged to, in a corresponding manner to the above,
c) determine whether during a time cycle said signal typical of an evoked response to a pacing pulse delivered by said second pacing means is sensed within a second time interval,
d) determine whether during the same time cycle said signal of the kind typical for an R-wave transferred from said first ventricle, or from some other part of the heart, to said second ventricle is detected within a second time window,
wherein the control circuit is arranged such that the operation of the device also depends on whether the conditions in c) and d) are fulfilled. Thereby the above described advantages are also achieved in connection with the second pacing and sensing means.

According to this embodiment, real loss can be distinguished from fusion also concerning the other ventricle. Also concerning this other ventricle, the control circuit can be arranged in a manner corresponding to the embodiments described above.

According to another aspect of the invention, the invention provides an implantable heart stimulating system comprising a device according to any of the above embodiments and a first and a second lead connected to said device, wherein said first pacing electrode is arranged on said first lead and said second pacing electrode is arranged on said second lead. Preferably, said first sensing electrode is the same electrode as said first pacing electrode and said second sensing electrode is the same electrode as said second pacing electrode. With such a system, the advantages described above are achieved.

### BRIEF DESCRIPTION OF THE DRAWINGS

- Fig 1: shows schematically a heart stimulating system with a heart stimulating device connected to leads with sensing and pacing electrodes positioned in a heart.
- Fig 2: shows schematically a control circuit which may form part of the device.
- Fig 3: shows schematically a somewhat more detailed illustration of part of the control circuit of Fig 2.
- Fig 4: shows schematically on a time scale signals related to first and second pacing and sensing means.

### DESCRIPTION OF PREFERRED EMBODIMENTS

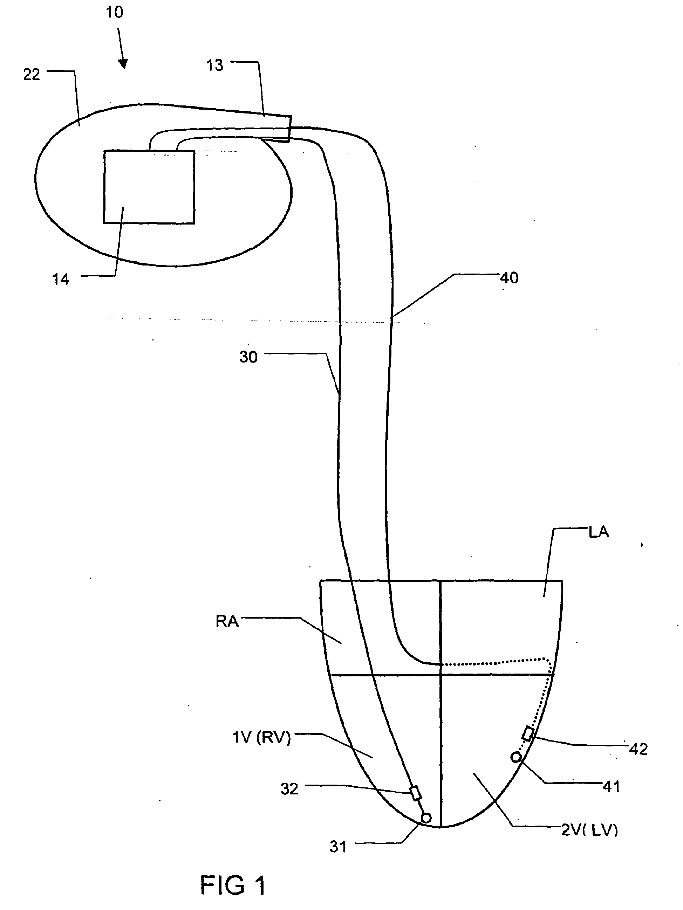

Fig 1 shows schematically an implantable heart stimulating device 10 according to the invention. The device 10 comprises a housing 22. The housing 22 includes a control circuit 14. The device 10 also comprises a connector portion 13. Via the connector portion 13, the device 10 can be connected to different leads. In Fig 1 the device 10 is connected to a first lead 30 and to a second lead 40. The device 10 together with the first 30 and the second 40 leads constitute an implantable heart stimulating system according to the invention. The first lead 30 includes a pacing and sensing electrode 31, 32. In this shown example this electrode 31, 32 is a bipolar electrode with a tip portion 31 and a ring portion 32. However, it is within of the scope of the invention that instead unipolar electrodes are used, as is known to a person skilled in the art. The second lead 40 has a corresponding electrode 41, 42.

Fig 1 also schematically illustrates a heart with a right atrium RA, a left atrium LA, a first ventricle 1V (which in this case is the right ventricle RV) and a second ventricle 2V (which in this case is the left ventricle LV). The electrode 31, 32 is positioned in the first ventricle 1V in order to be able to pace and sense this ventricle 1V. The electrode 41, 42 is positioned so as to pace and sense the second ventricle 2V. The second lead 40 may for example be introduced via the right atrium RA and the coronary sinus such that the electrode 41, 42 is positioned in for example the middle or great cardiac vein of the heart. How to introduce the lead 40 in this manner is known to a person skilled in the art. Although not shown in Fig 1, it is also possible that the system is connected to further leads with electrodes positioned in order to sense and/or pace the right atrium RA and the left atrium LA.

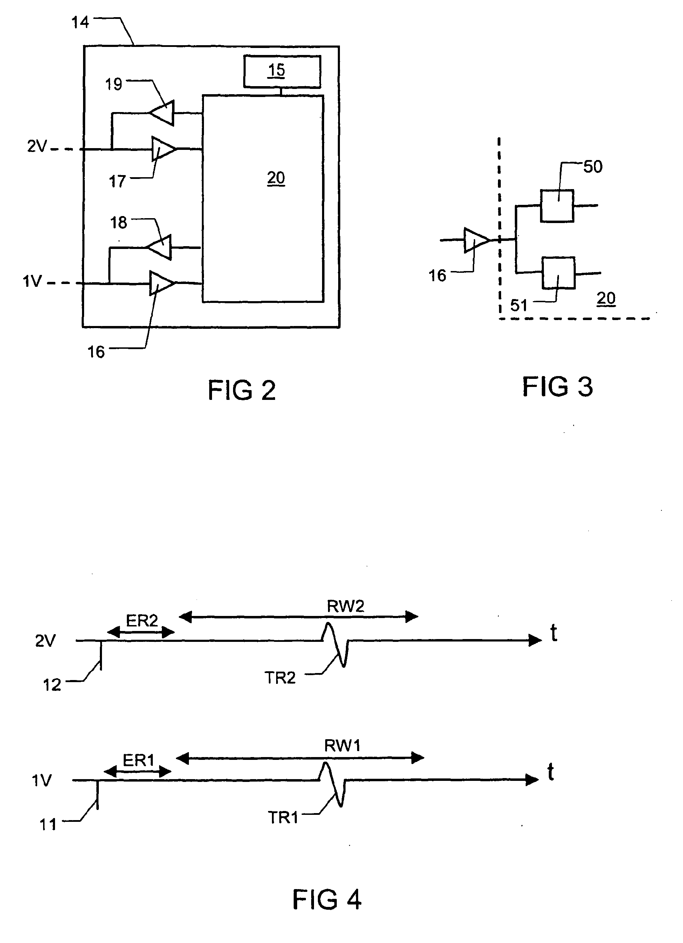

Fig 2 shows schematically the control circuit 14 in some more detail. The control circuit 14 includes a memory 15 connected to a control portion 20. The control circuit 14 comprises first pacing means 18. These means 18 are adapted to be connected to a first pacing electrode 31, 32, which, as shown in Fig 1, is positioned so as to receive signals from the first pacing means 18 such that the first pacing means 18 are able to pace the first ventricle 1V. The control circuit 14 also includes first sensing means 16. These means 16 are adapted to be connected to a first sensing electrode 31, 32, which can be positioned in the first ventricle 1V in order to transfer signals to the first sensing means 16. In this manner, the first sensing means 16 are able to sense the first ventricle 1 V. Although the first pacing electrode could be a different electrode from the first sensing electrode, it is preferred that the same electrode 31, 32 is used both for pacing and sensing.

The control circuit 14 also includes second pacing means 19 adapted to be connected to a second pacing electrode 41, 42 for pacing the second ventricle 2V of the heart. The control circuit 14 also includes second sensing means 17 adapted to be connected to a second sensing electrode 41, 42 in order to be able to sense the second ventricle 2V of the heart. The second pacing electrode is preferably the same electrode as the second sensing electrode. The control circuit 14 may of course also include means for pacing and sensing the atria of the heart.

Fig 4 shows schematically events related to the first ventricle 1 V and the second ventricle 2V on a time scale. The marker 11 represents a pacing pulse delivered by the first pacing means 18 and the marker 12 represents a pacing pulse delivered by the second pacing means 19.

The control circuit 14 and the first sensing means 16 are arranged to be able to detect a signal typical of an evoked response to a pacing pulse 11 delivered by the first pacing means 18 by sensing within a first time interval ER1.

According to a preferred embodiment, the control circuit 14 and the second sensing means 17 are also arranged to be able to detect a signal typical of an evoked response to the pacing pulse 12 delivered by the second pacing means 19 by sensing within a second time interval ER2. How to arrange the control circuit 14 in order to detect an evoked response is known to a person skilled in the art. The first time interval ER1 may for example be set to begin 15ms after the delivery of a pacing pulse 11 by the first pacing means 18. The length of the first time interval ER1 may for example be 50ms. Analogously, the second time interval ER2 may start 15ms after the delivery of a pacing pulse 12 by said second pacing means 19 and may have a length of about 50ms.

The control circuit 14 and the first sensing means 16 are also arranged to be able to detect, within a first time window RW1, a signal of the kind typical for an R-wave TR1 transferred from the second ventricle 2V, or from some other part of the heart, to the first ventricle 1V. The first time window RW1 is not identical with the first time interval ER1. As described above, if for example the first ventricle 1 V is depolarised, then this ventricle 1 V will be in the biological refractory period during for example about 350ms after the ventricle 1V was depolarised. During this biological refractory period, the first ventricle 1V cannot be depolarised again. However, in case the first ventricle 1V was not depolarised but the second ventricle 2V was depolarised, then, the depolarisation of the second ventricle 2V will via the myocardium reach the first ventricle 1 V and may be detected as a delayed depolarisation of the first ventricle 1 V. It is also possible that a transferred R-wave TR1can originate from some other part of the heart, such as from the A-V-node. Such a transferred R-wave TR1 can be detected within the mentioned first time window RW1. The time it takes for such a transferred R-wave TR1 to be sensed by the first sensing means 16 depends on the particular case. The control circuit 14 should therefore be arranged to define the first time window RW1 such that it is suitable for detecting a transferred R-wave TR1 in the particular case. The first time window RW1 may for example start 80ms after the delivery of a pacing pulse by the first pacing means 18. The first time window RW1 may for example be 200ms long. According to an alternative embodiment, the first time window RW1 can start directly after the delivery of a pacing pulse 11 by the first pacing means 18. However, according to a preferred embodiment the first time window RW1 does not start before the end of the first time interval ER1. It should be noted that in case the first time window RW1 coincides with the point in time when a pacing pulse, such as a back-up pulse, is delivered by the second pacing means 19, then the sensing of a transferred R-wave TR1 should preferably be disabled for a short time around such a point in time. In other words: the time window RW1 should in this case include a short blanking period during which the sensing of a transferred R-wave is not possible.

According to a preferred embodiment, the control circuit 14 and the second sensing means 17 are also arranged to be able to detect, within a second time window RW2, a signal of the kind typical for an R-wave TR2 transferred from the first ventricle 1 V, or from some other part of the heart, to the second ventricle 2V. The second time window RW2 is not identical with the second time interval ER2. Analogously to the description above, the second time window RW2 can for example start 80ms after the delivery of a pacing pulse by said second pacing means 19. The second time window RW2 may for example be 200ms long. Although it is possible that the second time window RW2 starts immediately after the delivery of a pacing pulse 12 by the second pacing means 19, according to a preferred embodiment, the second time window RW2 does not start before the end of the second time interval ER2. Also the second time window RW2 preferably includes a blanking period in case a back-up pulse is delivered by the first pacing means 18 during the second time window RW2.

It should be noted that the circuit arrangement for detecting an evoked response is preferably different from the arrangement for detecting other signals, such as a transferred R-wave TR1, TR2. Fig 3 shows schematically a part of the control circuit 14 in some more detail. Fig 3 illustrates that the first sensing means 16 is connected to an evoked response detection logic 50 and an R-wave detection logic 51. The detection logics 50 and 51 can be seen to form part of the control portion 20 illustrated in Fig 2. Preferably, similar detection logics are of course arranged also for the second sensing means 17. The detection logic 50 is thus optimised to sense an evoked response and the detection logic 51 optimised to detect an R-wave. The detection logic 50 is thus active during the first time interval ER1 and the detection logic 51 is active during the first time window RW1. It is possible that the first time window RW1 overlaps with the first time interval ER1 if the detection logics 50, 51 are sufficiently different to distinguish the different signals from each other. In particular, it is in this case important to be able to distinguish a transferred R-wave TR1 from a signal typical of a fusion beat. However, according to a preferred embodiment, the first time window RW1 does not overlap with the first time interval ER1. Analogously, the second time window RW2 does, according to one embodiment, not overlap with the second time interval ER2.

The control circuit 14 is arranged to be able to operate with time cycles corresponding to normal heart cycles. Such an operation is normal for an implantable heart stimulating device. The time cycle is determined by preset timer intervals which also may depend on detected signals.

The control circuit 14 can be arranged such that pacing pulses are delivered both by said first pacing means 18 and said second pacing means 19 during each time cycle. However, it is also possible that the control circuit 14 is arranged to inhibit the delivery of pacing pulses by said first pacing means 18 and/or said second pacing means 19 as is known to the person skilled in the art. Nevertheless, if pacing pulses are delivered both by said first pacing means 18 and said second pacing means 19 during a time cycle, then these pacing pulses are delivered at least substantially simultaneously. If this were not the case, but, for example, a pacing pulse 12 was delivered by the second pacing means 19 during the first time interval ER1, then it would be difficult to detect an evoked response to a pacing pulse 11 delivered by the first pacing means 18. The pacing pulses delivered by the first pacing means 18 and the second pacing means 19 should therefore be delivered at least substantially simultaneously such that the pacing pulses do not fall within the first or second time intervals ER1, ER2.

According to the invention, the control circuit is arranged to carry out the following steps:
a) determine whether during a time cycle a signal typical of an evoked response to a pacing pulse 11 delivered by the first pacing means 18 is sensed within the first time interval ER1,
b) determine whether during the same time cycle a signal typical of an R-wave TR1 transferred from the second ventricle 2V, or from some other part of the heart, to the first ventricle 1 V is detected within the first time window RW1. The control circuit 14 is arranged such that the operation of the device 10 depends on whether the conditions in a) and b) are fulfilled.

In case the condition in a) is not fulfilled and the condition in b) is fulfilled, this is an indication of a real loss of capture in the first ventricle 1 V. The control circuit 14 will thereby operate the device 10 in a manner L1. This manner L1 may for example comprise that an indication is stored in the memory 15 that a loss of capture in the first ventricle 1 V has been detected. When such a loss has been determined a predetermined number of times (which can be 1, 2 or any number of times), then the control circuit 14 can be arranged to vary the energy of the pacing pulses 11 delivered by the first pacing means 18 in order to perform a threshold search for finding a suitable energy for the pacing pulses. How to perform such a threshold search is known to a person skilled in the art and will therefore not be described in more detail here.

If, on the other hand, the condition in a) is not fulfilled and also the condition in b) is not fulfilled, then this is an indication of the fact that a fusion has probably occurred. The control circuit 14 is thereby arranged to operate the device 10 in another manner F1. This manner F1 may for example involve that an indication is stored in the memory 15 that such a fusion has been detected. Furthermore, in case such a fusion has been detected a predetermined number of times (which can be 1, 2 or any number of times) then the control circuit 14 can be arranged to modify a time period that controls the operation of the device 10. Depending on the operation mode of the device 10, the control circuit 14 can for example be arranged to increase the escape interval or to increase the A-V or P-V interval. This measure means that a possible stimulation pulse will be delivered later in time. Thereby the probability of a fusion decreases. If the device 10 is set to operate in an inhibition mode, this means that the intrinsic depolarisation is more likely to be sensed such that a pacing pulse can be inhibited.

It should also be noted that when the control circuit 14 is arranged to sense a possible transferred R-wave TR1 during the first time window RW1, then the control circuit 14 is preferably arranged such that no backup pulse is delivered during the time cycle in question, at least not by the first pacing means 18, since such a back-up pulse could interfere with a detection of the transferred R-wave TR1.

According to a preferred embodiment, the control circuit 14 is arranged to carry out steps corresponding to those described above also concerning the second ventricle 2V. In other words: the control circuit 14 is arranged to
c) determine whether during a time cycle a signal typical of an evoked response to a pacing pulse delivered by the second pacing means 19 is sensed within the second time interval ER2, and
d) determine whether during the same time cycle a signal typical for an R-wave TR2 transferred from the first ventricle 1 V, or from some other part of the heart, to the second ventricle 2V is detected within the second time window RW2. The control circuit 14 is arranged such that the operation of the device 10 depends on whether the conditions in c) and d) are fulfilled.

If the condition in c) is not fulfilled and the condition in d) is fulfilled, then the device is preferably arranged to operate in a manner L2, i. e. in a manner suitable if a real loss of capture has occurred in the second ventricle 2V. The device 10 can then be arranged to perform a threshold search of the same kind as has been described above in order to find a suitable pulse energy for the pacing pulses delivered by the second pacing means 19.

If the condition in c) is not fulfilled and the condition in d) is also not fulfilled, then the device 10 is preferably arranged to operate in another manner F2, i. e. a manner which is based on the assumption that a fusion has occurred in the second ventricle 2V. The control circuit 14 can thereby be arranged to operate such that a time period is increased in an analogous manner to the above description in connection with the first ventricle 1V.

The table below shows an example of decisions made by the device 10 on how to interpret the different signals detected and the pulses delivered.

| DETECTION | DECISION | |
|---|---|---|
| | FIRST | SECOND |
| | VENTRICLE | VENTRICLE |
| L1, C2, TR1, ― | Real loss | Capture |
| L1, C2, ― ,― | Fusion | Capture |
| C1, L2, ―, TR2 | Capture | Real loss |
| C1, L2, ― ,― | Capture | Fusion |
| i1, L2, ―, TR2 | Inhibition | Real loss |
| i1, L2, ―,― | Inhibition | Fusion |
| L1, i2, TR1, ― | Real loss | Inhibition |
| L1, i2, ―,― | Fusion | inhibition |
| i1, C2, ―, ― | Inhibition | Capture |
| C1, i2 , ―, ― | Capture | Inhibition |

In the table, 1 refers to the first ventricle 1V (and thereby to the first pacing means 18 and the first sensing means 16). 2 refers to the second ventricle 2V (and thereby to the second pacing means 19 and the second sensing means 17). L means that no evoked response is sensed during the respective time interval ER1 or ER2. C means on the other hand that an evoked response is sensed during said time interval ER1 or ER2. i refers to inhibition, i. e. that during the time cycle in question no pacing pulse has been delivered by the pacing means in question. In the first column (Detection) the first position thus refers to the first ventricle 1V and the second position refers to the second ventricle 2V. The third position concerns the detection of a signal typical for a transferred R-wave to the first ventricle 1V during the first time window RW1, i. e. the detection of a signal TR1. A hyphen (―) in this position means that no such signal TR1 has been detected. Analogously, the fourth position concerns the second ventricle 2V and the question whether a transferred signal TR2 is detected during the time window RW2.

The second and third columns in the table indicate how the signals are functionally interpreted by the device 10. In particular the difference between a real loss and a fusion as has been described above can be found in the table. The device 10 is thus arranged to operate in accordance with the decisions in the table. The person skilled in the art knows how the device is thereby suitably arranged to operate in response to the different situations such as real loss, fusion, capture and inhibition.

The system according to the invention can be used as follows. The system is implanted in a human or animal being and the first pacing electrode 31, 32 is positioned in or at the first ventricle 1V and the second pacing electrode 41, 42 is positioned in or at the second ventricle 2V as described above. Preferably, the system is used on a human or animal being suffering from congestive heart failure, for example caused by a bundle branch block.

## Claims

1. An implantable heart stimulating device (10) including a control circuit (14) comprising:
first pacing means (18) adapted to be connected to a first pacing electrode (31, 32) suited to be positioned in or at a first ventricle (1V) of a heart and to receive signals from said first pacing means (18) such that said first pacing means (18) are able to pace said first ventricle (1V);
first sensing means (16) adapted to be connected to a first sensing electrode (31, 32) suited to be positioned in or at said first ventricle (1V) of the heart and to transfer signals to said first sensing means (16) such that said first sensing means (16) are able to sense said first ventricle (1 V);
second pacing means (19) adapted to be connected to a second pacing electrode (41, 42) suited to be positioned in or at a second ventricle (2V) of the heart and to receive signals from said second pacing means (19) such that said second pacing means (19) are able to pace said second ventricle (2V);
second sensing means (17) adapted to be connected to a second sensing electrode (41, 42) suited to be positioned in or at said second ventricle (2V) of the heart and to transfer signals to said second sensing means (17) such that said second sensing means (17) are able to sense said second ventricle (2V);
said control circuit (14) and said first sensing means (16) being arranged to be able to detect a signal typical of an evoked response to a pacing pulse delivered by said first pacing means (18), by sensing within a first time interval (ER1) that follows after a pacing pulse delivered by said first pacing means (18);
said control circuit (14) and said first sensing means (16) also being arranged to be able to detect, within a first time window (RW1), a signal of the kind typical for an R-wave transferred from said second ventricle (2V), or from some other part of the heart, to said first ventricle (1V), wherein this first time window (RW1) is not identical with said first time interval (ER1),
said control circuit (14) being arranged to be able to operate with time cycles corresponding to normal heart cycles;
said control circuit (14) being arranged such that if within one such time cycle pacing pulses are delivered both by said first pacing means (18) and by said second pacing means (19), then these pacing pulses are delivered at least substantially simultaneously,
wherein the control circuit (14) is arranged to carry out the following steps:
a) determine whether during a time cycle said signal typical of an evoked response to a pacing pulse delivered by said first pacing means (18) is sensed within said first time interval (ER1),
b) determine whether during the same time cycle said signal of the kind typical for an R-wave transferred from said second ventricle (2V), of from some other part of the heart, to said first ventricle (1V) is detected within said first time window (RW1),
wherein the control circuit (14) is arranged such that the operation of the device (10) depends on whether the conditions in a) and b) are fulfilled.

2. An implantable heart stimulating device (10) according to claim 1, wherein the control circuit (14) is arranged such that the device (10) will operate in a manner L1 if it is the case both that the condition in a) is not fulfilled and that the condition in b) is fulfilled, and in another manner F1 if it is the case both that the condition in a) is not fulfilled and that the condition in b) is not fulfilled.

3. An implantable heart stimulating device (10) according to claim 2, wherein the control circuit (14) is arranged such that the manner L1 means that the device (10) functionally operates as if a real loss of capture has occurred in the first ventricle (1V) and such that the manner F1 means that the device (10) functionally operates as if a fusion has occurred in said first ventricle (1V).

4. An implantable heart stimulating device (10) according to claim 3, wherein the control circuit (14) is arranged such that the manner L1 comprises that at least if said case, that both the condition in a) is not fulfilled and that the condition in b) is fulfilled, has been determined a predetermined number of times, then the control circuit (14) is arranged to vary the energy of the pacing pulses delivered by said first pacing means (18) and to detect, with said first sensing (16) means, signals typical for evoked responses during said first time interval (ER1) such that a suitable pulse energy for the pacing pulses delivered by said first pacing means (18) is determined.

5. An implantable heart stimulating device (10) according to claim 3, wherein the control circuit (14) is arranged such that the manner F1 comprises that at least if said case, that both the condition in a) is not fulfilled and that the condition in b) is not fulfilled, has been determined a predetermined number of times, then the control circuit (14) is arranged to modify at least one time period that controls the operation of the device (10).

6. An implantable heart stimulating device (10) according to claim 5, wherein the modification of said time period means that the time period is increased or decreased.

7. An implantable heart stimulating device (10) according to any of the preceding claims, wherein the control circuit (14) is arranged such that said first time interval (ER1) starts 0-30ms after the delivery of a pacing pulse by said first pacing means (18) and is between 25ms and 100ms long.

8. An implantable heart stimulating device (10) according to any of the preceding claims, wherein the control circuit (14) is arranged such that said first time window (RW1) starts between 0 ms and 150 ms after the delivery of said pacing pulse by said first pacing means (18).

9. An implantable heart stimulating device (10) according to any of the preceding claims, wherein the control circuit (14) is arranged such that said first time window (RW1) ends at least before 400ms after the delivery of said pacing pulse by said first pacing means (18).

10. An implantable heart stimulating device (10) according to any of the preceding claims, wherein
said control circuit (14) and said second sensing means (17) are arranged to be able to detect a signal typical of an evoked response to a pacing pulse delivered by said second pacing means (19), by sensing within a second time interval (ER2) that follows after a pacing pulse delivered by said second pacing means (19); and wherein
said control circuit (14) and said second sensing means (17) are also arranged to be able to detect, within a second time window (RW2), a signal of the kind typical for an R-wave transferred from said first ventricle (1V), or from some other part of the heart, to said second ventricle (2V), wherein this second time window (RW2) is not identical with said second time interval (ER2),
wherein the control circuit (14) is arranged to carry out the following steps:
c) determine whether during a time cycle said signal typical of an evoked response to a pacing pulse delivered by said second pacing means (19) is sensed within said second time interval (ER2),
d) determine whether during the same time cycle said signal of the kind typical for an R-wave transferred from said first ventricle (1V), or from some other part of the heart, to said second ventricle (2V) is detected within said second time window (RW2),
wherein the control circuit (14) is arranged such that the operation of the device (10) also depends on whether the conditions in c) and d) are fulfilled.

11. An implantable heart stimulating device (10) according to claim 10, wherein the control circuit (14) is arranged such that the device (10) will operate in a manner L2 if it is the case both that the condition in c) is not fulfilled and that the condition in d) is fulfilled, and in another manner F2 if it is the case both that the condition in c) is not fulfilled and that the condition in d) is not fulfilled.

12. An implantable heart stimulating device (10) according to claim 11, wherein the control circuit (14) is arranged such that the manner L2 means that the device (10) functionally operates as if a real loss of capture has occurred in the second (2V) ventricle and such that the manner F2 means that the device (10) functionally operates as if a fusion has occurred in said second ventricle (2V).

13. An implantable heart stimulating device (10) according to claim 12, wherein the control circuit (14) is arranged such that the manner L2 comprises that at least if said case, that both the condition in c) is not fulfilled and that the condition in d) is fulfilled, has been determined a predetermined number of times, then the control circuit (14) is arranged to vary the energy of the pacing pulses delivered by said second pacing means (19) and to detect, with said second sensing means (17), signals typical for evoked responses during said second time interval (ER2) such that a suitable pulse energy for the pacing pulses delivered by said second pacing means (19) is determined.

14. An implantable heart stimulating device (10) according to claim 12, wherein the control circuit (14) is arranged such that the manner F2 comprises that at least if said case, that both the condition in c) is not fulfilled and that the condition in d) is not fulfilled, has been determined a predetermined number of times, then the control circuit (14) is arranged to modify at least one time period that controls the operation of the device (10).

15. An implantable heart stimulating device (10) according to claim 14, wherein the modification of said time period means that the time period is increased or decreased.

16. An implantable heart stimulating device (10) according to any of the claims 10-15, wherein the control circuit (14) is arranged such that said second time interval (ER2) starts 0-30ms after the delivery of a pacing pulse by said second pacing means (19) and is between 25ms and 100ms long.

17. An implantable heart stimulating device (10) according to any of the claims 10-16, wherein the control circuit (14) is arranged such that said second time window (RW2) starts between 0 ms and 150 ms after the delivery of said pacing pulse by said second pacing means (19).

18. An implantable heart stimulating device (10) according to any of the claims 10-17, wherein the control circuit (14) is arranged such that said second time window (RW2) ends at least before 400ms after the delivery of said pacing pulse by said second pacing means (19).

19. An implantable heart stimulating system comprising:
an implantable heart stimulating device (10) according to any of the preceding claims, and
a first (30) and a second (40) lead connected to said device (10), wherein said first pacing electrode (31, 32) is arranged on said first lead (30) and said second pacing electrode (41, 42) is arranged on said second lead.

20. An implantable heart stimulating system according to claim 19, wherein said first sensing electrode (31, 32) is the same electrode as said first pacing electrode (31, 32) and wherein said second sensing electrode (41, 42) is the same electrode as said second pacing electrode (41, 42).

## Patentansprüche

1. Implantierbare Herzstimulationsvorrichtung (10) mit einer Steuerschaltung (14), enthaltend:
eine erste Stimulationsvorrichtung (18), die ausgelegt ist, mit einer ersten Stimulationselektrode (31, 32) verbunden zu werden, welche geeignet ist, in oder an einem ersten Ventrikel (1V) eines Herzens positioniert zu werden und von der genannten ersten Stimulationsvorrichtung (18) Signale zu empfangen, so dass die genannte erste Stimulationsvorrichtung (18) fähig ist, den genannten ersten Ventrikel (1V) zu stimulieren;
eine erste Abfühlvorrichtung (16), die ausgelegt ist, mit einer ersten Abfühlelektrode (31, 32) verbunden zu werden, welche geeignet ist, in oder an dem genannten ersten Ventrikel (1V) des Herzens positioniert zu werden und zur genannten ersten Abfühlvorrichtung (16) Signale zu übertragen, so dass die genannte erste Abfühlvorrichtung (16) fähig ist, den genannten ersten Ventrikel (1V) abzufühlen;
eine zweite Stimulationsvorrichtung (19), die ausgelegt ist mit einer zweiten Stimulationselektrode (41, 42) verbunden zu werden, welche geeignet ist in oder an einem zweiten Ventrikel (2V) des Herzens positioniert zu werden und aus der genannten zweiten Stimulationsvorrichtung (19) Signale zu empfangen, so dass die genannte zweite Stimulationsvorrichtung (19) fähig ist, den genannten zweiten Ventrikel (2V) zu stimulieren;
eine zweite Abfühlvorrichtung (17), die ausgelegt ist, mit einer zweiten Abfühlelektrode (41, 42) verbunden zu werden, welche geeignet ist in oder an dem genannten zweiten Ventrikel (2V) des Herzens positioniert zu werden und zu der genannten zweiten Abfühlvorrichtung (17) Signale zu übertragen, so dass die genannte zweite Abfühlvorrichtung (17) fähig ist, den genannten zweiten Ventrikel (2V) abzufühlen;
wobei die genannte Steuerschaltung (14) und die genannte erste Abfühlvorrichtung (16) so ausgebildet sind, dass sie fähig sind, ein für eine evozierte Reaktion auf einen durch die genannte erste Stimulationsvorrichtung (18) ausgegebenen Stimulationsimpuls typisches Signal durch Abfühlen innerhalb eines ersten Zeitintervalls (ER1) zu detektieren, das auf einen Stimulationsimpuls folgt, der durch die genannte erste Stimulationsvorrichtung (18) ausgegeben worden ist;
die genannte Steuerschaltung (14) und die genannte erste Abfühlvorrichtung (16) außerdem so ausgebildet sind, dass sie fähig sind, innerhalb eines ersten Zeitfensters (RW1) ein Signal zu detektieren, das typisch für die Art einer, von dem genannten zweiten Ventrikel (2V) oder von irgendeinem anderen Teil des Herzens zu dem genannte ersten Ventrikel (1V) übertragenen R-Welle ist, wobei dieses erste Zeitfenster (RW1) nicht identisch mit dem genannten ersten Zeitintervall (ER1) ist,
die genannte Steuervorrichtung (14) so ausgebildet ist, dass sie fähig ist, mit Zeitzyklen zu arbeiten, die normalen Herzzyklen entsprechen;
die genannte Steuervorrichtung (14) so ausgebildet ist, dass, falls innerhalb eines solchen Zeitzyklus Stimulationsimpulse sowohl durch die genannte erste Stimulationsvorrichtung (18) als auch durch die genannte zweite Stimulationsvorrichtung (19) geliefert werden, diese Stimulationsimpulse dann im wesentlichen gleichzeitig geliefert werden,
wobei die Steuervorrichtung (14) so ausgebildet ist, dass sie die folgenden Schritte ausführt:
a) Bestimme, ob während eines Zeitzyklus das genannte Signal, das typisch für eine evozierte Reaktion auf einen durch die genannte erste Stimulationsvorrichtung (18) gelieferten Stimulationsimpuls ist, innerhalb des genannten ersten Zeitintervalls (ER1) abgefühlt wird,
b) Bestimme, ob während des selben Zeitzyklus das genannte Signal von der Art, die typisch für eine von dem genannten zweiten Ventrikel (2V) oder von irgendeinem anderen Teil des Herzens zu dem genannten ersten Ventrikel (1 V) übertragene R-Welle ist, innerhalb des genannten ersten Zeitfensters (RW1) detektiert wird,
wobei die Steuerschaltung (14) so ausgebildet ist, dass die Arbeitsweise der Vorrichtung (10), davon abhängt, ob die Bedingungen von a) und b) erfüllt werden.

2. Implantierbare Herzstimulationsvorrichtung (10) nach Anspruch 1, wobei die Steuerschaltung (14) so ausgebildet ist, dass die Vorrichtung (10) in einer Arbeitsweise L1 arbeitet, falls die Bedingung von a) nicht erfüllt ist und die Bedingung von b) erfüllt ist, und in einer anderen Arbeitsweise F1, falls die Bedingung von a) nicht erfüllt ist und die Bedingung von b) nicht erfüllt ist.

3. Implantierbare Herzstimulationsvorrichtung (10) nach Anspruch 2, wobei die Steuerschaltung (14) so ausgebildet ist, dass die Arbeitsweise L1 bedeutet, dass die Vorrichtung (10) funktionell so arbeitet, wie wenn im ersten Ventrikel (1V) ein realer Captureverlust aufgetreten wäre und so ausgebildet ist, dass die Arbeitsweise F1 bedeutet, dass die Vorrichtung (10) funktionell so arbeitet, wie wenn in dem genannten ersten Ventrikel (1V) eine Fusion aufgetreten wäre.

4. Implantierbare Herzstimulationsvorrichtung (10) nach Anspruch 3, wobei die Steuerschaltung (14) so ausgebildet ist, dass die Arbeitsweise L1 umfasst, dass, wenn der genannte Fall, dass die Bedingung von a) nicht erfüllt ist und die Bedingung von b) erfüllt ist wenigstens eine vorbestimmte Anzahl von Malen bestimmt worden ist, die Steuerschaltung (14) so ausgebildet ist, dass sie dann die Energie der durch die genannte erste Stimulationsvorrichtung (18) gelieferten Stimulationsimpulse variiert und mit der genannten ersten Abfühlvorrichtung (16) die Signale erfasst, die typisch für evozierte Reaktionen während des genannten ersten Zeitintervalls (ER1) sind, derart, dass für die durch die genannte erste Stimulationsvorrichtung (18) gelieferten Stimulationsimpulse eine geeignete Impulsenergie bestimmt wird.

5. Implantierbare Herzstimulationsvorrichtung (10) nach Anspruch 3, wobei die Steuerschaltung (14) so ausgebildet ist, dass die Arbeitsweise F1 umfasst, dass wenn der genannte Fall, dass die Bedingung von a) nicht erfüllt ist und dass die Bedingung von b) nicht erfüllt ist, wenigstens eine vorbestimmte Anzahl von Malen bestimmt worden ist, die Steuerschaltung (14) ausgebildet ist, dann wenigstens eine Zeitperiode zu modifizieren, die die Arbeitsweise der Vorrichtung (10) steuert.

6. Implantierbare Herzstimulationsvorrichtung (10) nach Anspruch 5, wobei die Modifikation der genannten Zeitperiode bedeutet, dass die Zeitperiode vergrößert oder verkleinert wird.

7. Implantierbare Herzstimulationsvorrichtung (10) nach einem der vorhergehenden Ansprüche, wobei die Steuerschaltung (14) so ausgebildet ist, dass das genannte erste Zeitintervall (ER1) 0-30ms nach der Ausgabe eines Stimulationsimpulses durch die genannte erste Stimulationsvorrichtung (18) beginnt und zwischen 25ms und 100ms lang ist.

8. Implantierbare Herzstimulationsvorrichtung (10) nach einem der vorhergehenden Ansprüche, wobei die Steuerschaltung (14) so ausgebildet ist, dass das genannte erste Zeitfenster (RW1) zwischen 0ms und 150ms nach der Ausgabe des genannten Stimulationsimpulses durch die genannte erste Stimulationsvorrichtung (18) beginnt.

9. Implantierbare Herzstimulationsvorrichtung (10) nach einem der vorhergehenden Ansprüche, wobei die Steuerschaltung (14) so ausgebildet ist, dass das genannte erste Zeitfenster (RW1) wenigstens vor Ablauf von 400ms nach der Ausgabe des genannten Stimulationsimpulses durch die genannte erste Stimulationsvorrichtung (18) endet.

10. Implantierbare Herzstimulationsvorrichtung (10) nach einem der vorhergehenden Ansprüche, wobei
die genannte Steuerschaltung (14) und die genannte zweite Abfühlvorrichtung (17) so ausgebildet sind, dass sie befähigt sind, ein Signal zu erfassen, das typisch für eine evozierte Reaktion auf einen durch die genannte zweite Stimulationsvorrichtung (19) gelieferten Stimulationsimpuls ist, durch Abfühlen innerhalb eines zweiten Zeitintervalls (ER2) das auf einen, durch die genannte zweite Stimulationsvorrichtung (19) gelieferten Stimulationsimpuls folgt;
und wobei
die genannte Steuerschaltung (14) und die genannte zweite Abfühlvorrichtung (17) so ausgebildet sind, dass sie fähig sind, innerhalb eines zweiten Zeitfensters (RW2) ein Signal einer Art zu detektieren, die typisch für eine von dem genannten ersten Ventrikel (1V) oder von irgend einem anderen Teil des Herzens zu dem genannten zweiten Ventrikel (2V) übertragene R-Welle ist, wobei dieses zweite Zeitfenster (RW2) nicht mit dem genannten zweiten Zeitintervall (ER2) identisch ist,
wobei die Steuerschaltung (14) so ausgebildet ist, dass sie die folgenden Schritte ausführt:
c) bestimme ob während eines Zeitzyklus das genannte Signal, das typisch für eine evozierte Reaktion auf einen durch die genannte zweite Stimulationsvorrichtung (19) gelieferten Stimulationsimpuls ist, innerhalb des genannten zweiten Zeitintervalls (ER2) abgefühlt wird,
d) bestimme, ob während des selben Zeitzyklus das genannte Signal von der Art, die typisch für eine von dem genannten ersten Ventrikel (1V) oder von irgend einem anderen Teil des Herzens zu dem genannten zweiten Ventrikel (2V) übertragene R-Welle ist, innerhalb des genannten zweiten Zeitfensters (RW2) detektiert wird,
wobei die Steuerschaltung (14) so ausgebildet ist, dass die Arbeitsweise der Vorrichtung (10) davon abhängt, ob die Bedingungen von c) und d) erfüllt werden.

11. Implantierbare Herzstimulationsvorrichtung (10) nach Anspruch 1, wobei die Steuerschaltung (14) so ausgebildet ist, dass die Vorrichtung (10) in einer Arbeitsweise L2 arbeitet, falls die Bedingung von c) nicht erfüllt ist und die Bedingung von d) erfüllt ist, und in einer anderen Arbeitsweise F2, falls die Bedingung von c) nicht erfüllt ist und die Bedingung von d) nicht erfüllt ist.

12. Implantierbare Herzstimulationsvorrichtung (10) nach Anspruch 11, wobei die Steuerschaltung (14) so ausgebildet ist, dass die Arbeitsweise L2 bedeutet, dass die Vorrichtung (10) funktionell so arbeitet, wie wenn im zweiten Ventrikel (2V) ein realer Captureverlust aufgetreten wäre und so ausgebildet ist, dass die Arbeitsweise F2 bedeutet, dass die Vorrichtung (10) funktionell so arbeitet, wie wenn in dem genannten zweiten Ventrikel (2V) eine Fusion aufgetreten wäre

13. Implantierbare Herzstimulationsvorrichtung (10) nach Anspruch 12, wobei die Steuerschaltung (14) so ausgebildet ist, dass die Arbeitsweise L2 umfasst, dass, wenn der genannte Fall, dass die Bedingung von c) nicht erfüllt ist und die Bedingung von d) erfüllt ist, wenigstens eine vorbestimmte Anzahl von Malen bestimmt worden ist, die Steuerschaltung (14) so ausgebildet ist, dass sie dann die Energie der durch die genannte zweite Stimulationsvorrichtung (19) gelieferten Stimulationsimpulse variiert und mit der genannten zweiten Abfühlvorrichtung (17) die Signale erfasst, die typisch für evozierte Reaktionen während des genannten zweiten Zeitintervalls (ER2) sind, derart, dass für die durch die genannte zweite Stimulationsvorrichtung (19) gelieferten Stimulationsimpulse eine geeignete Impulsenergie bestimmt wird.

14. Implantierbare Herzstimulationsvorrichtung (10) nach Anspruch 12, wobei die Steuerschaltung (14) so ausgebildet ist, dass die Arbeitsweise F2 umfasst, dass, wenn der genannte Fall, dass die Bedingung von c) nicht erfüllt ist und dass die Bedingung von d) nicht erfüllt ist, wenigstens eine vorbestimmte Anzahl von Malen bestimmt worden ist, die Steuerschaltung (14) ausgebildet ist, dann wenigstens eine Zeitperiode zu modifizieren, die die Arbeitsweise der Vorrichtung (10) steuert.

15. Implantierbare Herzstimulationsvorrichtung (10) nach Anspruch 14, wobei die Modifikation der genannten Zeitperiode bedeutet, dass die Zeitperiode vergrößert oder verkleinert wird.

16. Implantierbare Herzstimulationsvorrichtung (10) nach einem der vorhergehenden Ansprüche, wobei die Steuerschaltung (14) so ausgebildet ist, dass das genannte zweite Zeitintervall (ER2) 0-30ms nach der Ausgabe eines Stimulationsimpulses durch die genannte zweite Stimulationsvorrichtung (19) beginnt und zwischen 25ms und 100ms lang ist.

17. Implantierbare Herzstimulationsvorrichtung (10) nach einem der vorhergehenden Ansprüche, wobei die Steuerschaltung (14) so ausgebildet ist, dass das genannte zweite Zeitfenster (RW2) zwischen 0ms und 150ms nach der Ausgabe des genannten Stimulationsimpulses durch die genannte zweite Stimulationsvorrichtung (19) beginnt.

18. Implantierbare Herzstimulationsvorrichtung (10) nach einem der Ansprüche 10 bis 17, wobei die Steuerschaltung (14) so ausgebildet ist, dass das genannte zweite Zeitfenster (RW2) wenigstens vor Ablauf von 400ms nach der Ausgabe des genannten Stimulationsimpulses durch die genannte zweite Stimulationsvorrichtung (19) endet.

19. Implantierbares Herzstimulationssystem, enthaltend:
eine implantierbare Herzstimulationsvorrichtung (10) nach einem der vorhergehenden Ansprüche und
eine erste (30) und eine zweite (40) mit der genannten Vorrichtung (10) verbundene Leitung, wobei die genannte erste Stimulationselektrode (31, 32) an der genannten ersten Leitung (30) angeordnet ist und die genannte zweite Stimulationselektrode (41, 42) an der genannten zweiten Leitung angeordnet ist.

20. Implantierbares Herzstimulationssystem nach Anspruch 19, wobei die genannte erste Abfühlelektrode (31, 32) die selbe Elektrode wie die genannte erste Stimulationselektrode (31, 32) ist und wobei die genannte zweite Abfühlelektrode (41, 42) die selbe Elektrode wie die genannte zweite Stimulationselektrode (41, 42) ist.

## Revendications

1. Dispositif (10) implantable de stimulation cardiaque comportant un circuit (14) de commande comprenant :
des premiers moyens (18) de stimulation agencés pour être connectés à une première électrode (31, 32) de stimulation conçue pour être positionnée dans ou au niveau d'un premier ventricule (1V) d'un coeur et pour recevoir des signaux en provenance des premiers moyens (18) de stimulation, de manière à ce que les premiers moyens (18) de stimulation puissent stimuler le premier ventricule (1V) ;
des premiers moyens (16) de détection agencés pour être connectés à une première électrode (31, 32) de détection conçue pour être positionnée dans ou au niveau du premier ventricule (1V) du coeur et pour transférer des signaux aux premiers moyens (16) de détection, de manière à ce que les premiers moyens (16) de détection puissent détecter le premier ventricule (1V) ;
des deuxièmes moyens (19) de stimulation agencés pour être connectés à une deuxième électrode (41, 42) de stimulation conçue pour être positionnée dans ou au niveau d'un deuxième ventricule (2V) du coeur et pour recevoir des signaux en provenance des deuxièmes moyens (19) de stimulation, de manière à ce que les deuxièmes moyens (19) de stimulation puissent stimuler le deuxième ventricule (2V) ;
des deuxièmes moyens (17) de détection agencés pour être connectés à une deuxième électrode (41, 42) de détection conçue pour être positionnée dans ou au niveau du deuxième ventricule (2V) du coeur et pour transférer des signaux aux deuxièmes moyens (17) de détection, de manière à ce que les deuxièmes moyens (17) de détection puissent détecter le deuxième ventricule (2V) ;
le circuit (14) de commande et les premiers moyens (16) de détection étant conçus pour pouvoir détecter un signal caractéristique d'une réponse évoquée à une impulsion de stimulation délivrée par les premiers moyens (18) de stimulation, en effectuant la détection à l'intérieur d'un premier intervalle (ER1) de temps qui fait suite à une impulsion de stimulation délivrée par les premiers moyens (18) de stimulation ;
le circuit (14) de commande et les premiers moyens (16) de détection étant également conçus pour pouvoir détecter, à l'intérieur d'une première fenêtre (RW1) de temps, un signal du type caractéristique d'une onde R, transféré du deuxième ventricule (2V), ou d'une autre partie du coeur, au premier ventricule (1V), cette première fenêtre (RW1) de temps n'étant pas identique au premier intervalle (ER1) de temps,
le circuit (14) de commande étant conçu pour pouvoir fonctionner suivant des cycles de temps correspondant à des cycles cardiaques normaux ;
le circuit (14) de commande étant conçu de manière à ce que si, à l'intérieur de l'un des cycles de temps, des impulsions de stimulation sont délivrées à la fois par les premiers moyens (18) de stimulation et par les deuxièmes moyens (19) de stimulation, ces impulsions de stimulation sont délivrées au moins sensiblement simultanément ;
dans lequel le circuit (14) de commande est conçu pour exécuter les étapes suivantes :
a) déterminer si, au cours d'un cycle de temps, le signal caractéristique d'une réponse évoquée à une impulsion de stimulation, délivrée par les premiers moyens (18) de stimulation, est détecté à l'intérieur du premier intervalle (ER1) de temps,
b) déterminer si, au cours du même cycle de temps, le signal du type caractéristique d'une onde R transféré du deuxième ventricule (2V), ou d'une autre partie du coeur, au premier ventricule (1V), est détecté à l'intérieur de la première fenêtre (RW1) de temps,
dans lequel le circuit (14) de commande est conçu de manière à ce que le fonctionnement du dispositif (10) soit fonction du fait que les conditions en a) et b) soient remplies ou non.

2. Dispositif (10) implantable de stimulation cardiaque, suivant la revendication 1, dans lequel le circuit (14) de commande est conçu de façon à ce que le dispositif (10) fonctionne d'une manière L1, dans le cas où à la fois la condition en a) n'est pas remplie et la condition en b) est remplie, et d'une autre manière F1, dans le cas où à la fois la condition en a) n'est pas remplie et la condition en b) n'est pas remplie.

3. Dispositif (10) implantable de stimulation cardiaque, suivant la revendication 2, dans lequel le circuit (14) de commande est conçu de telle sorte que la manière L1 signifie que le dispositif (10) fonctionne, d'un point de vue fonctionnel, comme si une perte réelle de capture s'était produite dans le premier ventricule (1V) et de telle sorte que la manière F1 signifie que le dispositif (10) fonctionne, d'un point de vue fonctionnel, comme si une fusion s'était produite dans le premier ventricule (1V).

4. Dispositif (10) implantable de stimulation cardiaque, suivant la revendication 3, dans lequel le circuit (14) de commande est conçu de telle sorte que la manière L1 comprend le fait qu'au moins si le cas où à la fois la condition en a) n'est pas remplie et la condition en b) est remplie, a été déterminé un nombre de fois déterminé à l'avance, le circuit (14) de commande est conçu pour modifier l'énergie des impulsions de stimulation délivrées par les premiers moyens (18) de stimulation et pour détecter, à l'aide des premiers moyens (16) de détection, des signaux caractéristiques de réponses évoquées, au cours du premier intervalle (ER1) de temps, afin qu'une énergie d'impulsion appropriée des impulsions de stimulation délivrées par les premiers moyens (18) de stimulation soit déterminée.

5. Dispositif (10) implantable de stimulation cardiaque, suivant la revendication 3, dans lequel le circuit (14) de commande est conçu de telle sorte que la manière F1 comprend le fait qu'au moins si le cas où à la fois la condition en a) n'est pas remplie et la condition en b) n'est pas remplie, a été déterminé un nombre de fois déterminé à l'avance, le circuit (14) de commande est conçu pour modifier au moins une période temporelle qui régit le fonctionnement du dispositif (10).

6. Dispositif (10) implantable de stimulation cardiaque suivant la revendication 5, dans lequel la modification de la période temporelle signifie que la période temporelle est augmentée ou diminuée.

7. Dispositif (10) implantable de stimulation cardiaque, suivant l'une quelconque des revendications précédentes, dans lequel le circuit (14) de commande est conçu de telle sorte que le premier intervalle (ER1) de temps débute 0 à 30 ms après la délivrance d'une impulsion de stimulation par les premiers moyens (18) de stimulation et a une durée comprise entre 25 ms et 100 ms.

8. Dispositif (10) implantable de stimulation cardiaque, suivant l'une quelconque des revendications précédentes, dans lequel le circuit (14) de commande est conçu de telle sorte que la première fenêtre (RW1) de temps débute entre 0 ms à 150 ms après la délivrance de l'impulsion de stimulation par les premiers moyens (18) de stimulation.

9. Dispositif (10) implantable de stimulation cardiaque, suivant l'une quelconque des revendications précédentes, dans lequel le circuit (14) de commande est conçu de telle sorte que la première fenêtre (RW1) de temps prend fin au moins avant 400 ms après la délivrance de l'impulsion de stimulation par les premiers moyens (18) de stimulation.

10. Dispositif (10) implantable de stimulation cardiaque, suivant l'une quelconque des revendications précédentes, dans lequel
le circuit (14) de commande et les deuxièmes moyens (17) de détection sont conçus pour pouvoir détecter un signal caractéristique d'une réponse évoquée à une impulsion de stimulation délivrée par les deuxièmes moyens (19) de stimulation, en effectuant la détection à l'intérieur d'un deuxième intervalle (ER2) de temps qui fait suite à une impulsion de stimulation délivrée par les deuxièmes moyens (19) de stimulation ; et dans lequel
le circuit (14) de commande et les deuxièmes moyens (17) de détection sont également conçus pour pouvoir détecter, à l'intérieur d'une deuxième fenêtre (RW2) de temps, un signal du type caractéristique d'une onde R, transféré du premier ventricule (1V), ou d'une autre partie du coeur, au deuxième ventricule (2V), cette deuxième fenêtre (RW2) de temps n'étant pas identique au deuxième intervalle (ER2) de temps,
dans lequel le circuit (14) de commande est conçu pour exécuter les étapes suivantes :
c) déterminer si, au cours d'un cycle de temps, le signal caractéristique d'une réponse évoquée à une impulsion de stimulation délivrée par les deuxièmes moyens (19) de stimulation, est détecté à l'intérieur du deuxième intervalle (ER2) de temps,
d) déterminer si, au cours du même cycle de temps, le signal du type caractéristique d'une onde R transféré du premier ventricule (1V), ou d'une autre partie du coeur, au deuxième ventricule (2V), est détecté à l'intérieur de la deuxième fenêtre (RW2) de temps,
dans lequel le circuit (14) de commande est conçu de manière à ce que le fonctionnement du dispositif (10) soit également fonction du fait que les conditions en c) et d) soient remplies ou non.

11. Dispositif (10) implantable de stimulation cardiaque, suivant la revendication 10, dans lequel le circuit (14) de commande est conçu de façon à ce que le dispositif (10) fonctionne d'une manière L2, dans le cas où à la fois la condition en c) n'est pas remplie et la condition en d) est remplie, et d'une autre manière F2, dans le cas où à la fois la condition en c) n'est pas remplie et la condition en d) n'est pas remplie.

12. Dispositif (10) implantable de stimulation cardiaque, suivant la revendication 11, dans lequel le circuit (14) de commande est conçu de telle sorte que la manière L2 signifie que le dispositif (10) fonctionne, d'un point de vue fonctionnel, comme si une perte réelle de capture s'était produite dans le deuxième ventricule (2V) et de telle sorte que la manière F2 signifie que le dispositif (10) fonctionne, d'un point de vue fonctionnel, comme si une fusion s'était produite dans le deuxième ventricule (2V).

13. Dispositif (10) implantable de stimulation cardiaque, suivant la revendication 12, dans lequel le circuit (14) de commande est conçu de telle sorte que la manière L2 comprend le fait qu'au moins si le cas où à la fois la condition en c) n'est pas remplie et la condition en d) est remplie, a été déterminé un nombre de fois déterminé à l'avance, le circuit (14) de commande est conçu pour modifier l'énergie des impulsions de stimulation délivrées par les deuxièmes moyens (19) de stimulation et pour détecter, à l'aide des deuxièmes moyens (17) de détection, des signaux caractéristiques de réponses évoquées, au cours du deuxième intervalle (ER2) de temps, afin qu'une énergie d'impulsion appropriée des impulsions de stimulation délivrées par les deuxièmes moyens (19) de stimulation soit déterminée.

14. Dispositif (10) implantable de stimulation cardiaque, suivant la revendication 12, dans lequel le circuit (14) de commande est conçu de telle sorte que la manière F2 comprend le fait qu'au moins si le cas où à la fois la condition en c) n'est pas remplie et la condition en d) n'est pas remplie, a été déterminé un nombre de fois déterminé à l'avance, le circuit (14) de commande est conçu pour modifier au moins une période temporelle qui régit le fonctionnement du dispositif (10).

15. Dispositif (10) implantable de stimulation cardiaque suivant la revendication 14, dans lequel la modification de la période temporelle signifie que la période temporelle est augmentée ou diminuée.

16. Dispositif (10) implantable de stimulation cardiaque, suivant l'une quelconque des revendications 10 à 15, dans lequel le circuit (14) de commande est conçu de telle sorte que le deuxième intervalle (ER2) de temps débute 0 à 30 ms après la délivrance d'une impulsion de stimulation par les deuxièmes moyens (19) de stimulation et a une durée comprise entre 25 ms et 100 ms.

17. Dispositif (10) implantable de stimulation cardiaque, suivant l'une quelconque des revendications 10 à 16, dans lequel le circuit (14) de commande est conçu de telle sorte que la deuxième fenêtre (RW2) de temps débute entre 0 ms à 150 ms après la délivrance de l'impulsion de stimulation par les deuxièmes moyens (19) de stimulation.

18. Dispositif (10) implantable de stimulation cardiaque, suivant l'une quelconque des revendications 10 à 17, dans lequel le circuit (14) de commande est conçu de telle sorte que la deuxième fenêtre (RW2) de temps prend fin au moins avant 400 ms après la délivrance de l'impulsion de stimulation par les deuxièmes moyens (19) de stimulation.

19. Système implantable de stimulation cardiaque, comprenant :
un dispositif (10) implantable de stimulation cardiaque, suivant l'une quelconque des revendications précédentes, et
des première (30) et deuxième (40) dérivations connectées au dispositif (10), la première électrode (31, 32) de stimulation étant placée sur la première dérivation (30) et la deuxième électrode (41, 42) de stimulation étant placée sur la deuxième dérivation.

20. Système implantable de stimulation cardiaque, suivant la revendication 19, dans lequel la première électrode (31, 32) de détection est la même électrode que la première électrode (31, 32) de stimulation et dans lequel la deuxième électrode (41, 42) de détection est la même électrode que la deuxième électrode (41, 42) de stimulation.
